# EUROPEAN PATENT SPECIFICATION

(11) **EP 0 504 529 B1**
(45) Date of publication and mention of the grant of the patent: **08.03.1995**
(21) Application number: 91311155.5
(22) Date of filing: 29.11.1991
(51) Int. Cl.: B25G 3/26, G01N 1/06, B01L 9/00

(54) **Holder for clamping used microtome blade**
Halter zum Festklemmen gebrauchter Mikrotommesser
Support de fixation pour lame de microtome usée

(30) Priority: 18.03.1991 JP 23369/91
(43) Date of publication of application: 23.09.1992
(73) Proprietor: KABUSHIKI KAISHA CHIBA MEDIKARU, Souka-shi, Saitama-ken (JP)
(72) Inventor: Chiba, Tutomu, Souka-shi, Saitama-ken (JP)
(74) Representative: Gura, Henry Alan

(56) References cited:
- GB-A- 158 044
- US-A- 4 137 631
- US-A- 4 207 790
- US-A- 4 400 878
- US-A- 4 617 736

## Description

The present invention relates to a holder for clamping used microtome blade, in more particular to a holder which enables to safely reuse a used microtome blade as a trimming cutter or as a stationary cutter for cutting paper or the like.

A microtome is exclusively used for preparing sliced thin samples to be examined with an optical or an electron microscope for observing tissue of a human body, animal or the like for the purpose of examination, studies and researches or the like in the field of medical science, the zoology, the botany or the like.

These microtome blades have usually been discarded whenever its sharpness has slightly degraded, since no more proper tissue samples having a smooth surface required for above-mentioned microscopic examination can be obtained by such a used and degraded microtome blade.

However, microtome blades whose sharpness has somewhat degraded by tissue slicing as mentioned above still have a sharpness sufficient for reusing them as a trimming knife for medical use or as a stationary knife or the like. Accordingly, there has been a strong demand for reusing such used microtome blades. However, since the length of these microtome blades are relatively long in as compared with the width and thickness thereof and they have still a certain degree of sharpness, direct handling of them in the fingers is difficult and dangerous.

US 4,137,631 discloses a holder for disposable blades in which the blade is clamped at the front end of a bar-like body by a thumbscrew. The ends of the blade are notched in order to embrace a pin projecting into the slit, the provision of the notches being necessary to lock the inserted blade against movement when pressure is applied to the blade to make a cut.

According to the present invention, a holder for clamping a microtome blade comprises an elongate body, a front end of the body being bifurcated by a longitudinal slit and the bifurcated parts of the front end of the body being provided with means for clamping them onto a microtome blade inserted in said slit, an inclined bore extending transversely through the body and terminating at said through-bore, the rear wall of said bore serving as a contacting location transverse to the body for the rear end of a blade inserted in said slit.

The holder of the present invention is well adapted for use with microtome blades that have chamfered corners. The blade can be held surely and safely while it is prevented from accidentally rotating in its own plane. It is not necessary to provide the blade with a notch.

The foregoing and other features and attendant advantages of the present invention will be appreciated as the same become better understood by means of the following description and accompanying drawings wherein:
Fig. 1 is a top view illustrating a holder for used microtome blades, in one embodiment of the present invention;
Fig. 2 is a sectional view along line II-II in Fig. 1;
Fig. 3 is a top view illustrating a holder for microtome blades in a second embodiment of the present invention;
Fig. 4 is a sectional view along line IV-IV in Fig. 3;
Fig. 5 is a sectional view similar to Fig. 3, illustrating a variant form of the holder shown in Fig. 3.

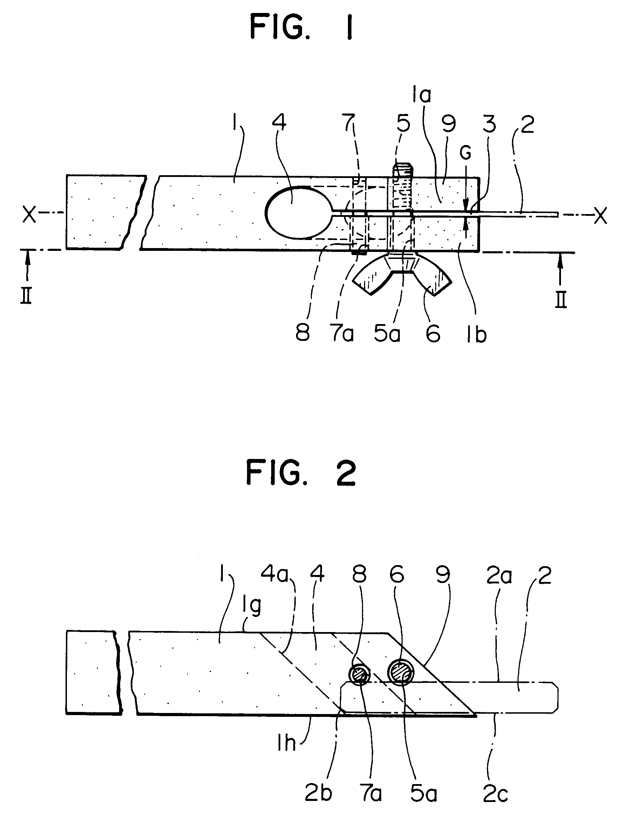

Referring now to Figs. 1 and 2 which show a holder for clamping used microtome blade, a plastic barlike holder body 1 having a rectangular cross-sectional shape is cut in its front end portion so as to be formed with a slit 3 in a plane substantially along the longitudinal axis X-X thereof. That is, the front end portion of the holder body 1 is bifurcated into two half parts 1a and 1b. The front face 9 of this front end portion is inclined, at which the front end of the slit 3 is opened to the outside. The slit 3 extends through the front end portion, entirely in the height-wise direction. Further, a through-bore 4 is formed in the front end portion so as to extend at a slant from the top face 1g to the bottom face 1h of the holder substantially parallel to the inclined front end face 9 and in the plane in which the slit 3 is formed so that the slit 3 is terminated by this through-bore 4.

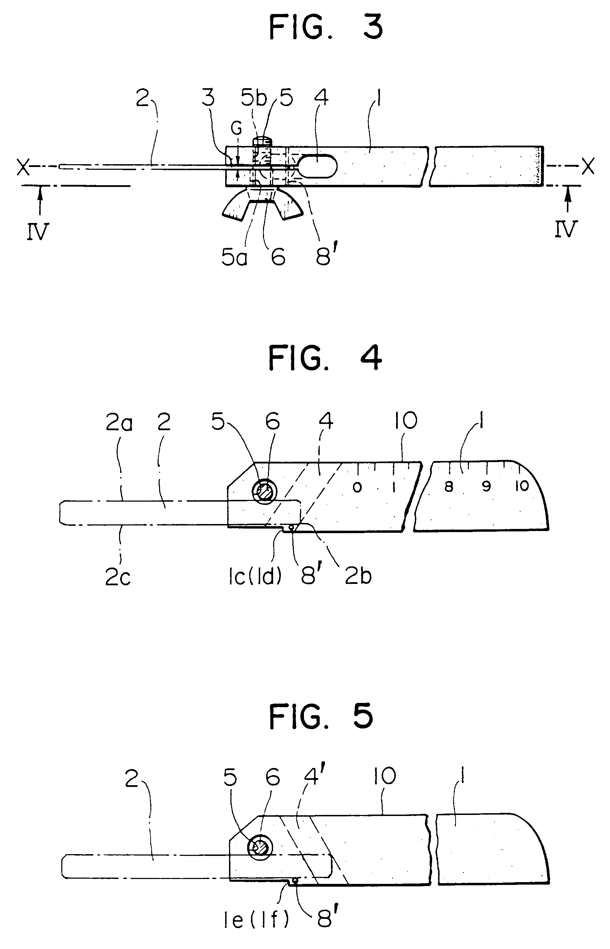

It is to be noted that the through-bore 4 apparently has an elliptic shape as seen at the top face 1g and the bottom face 1h of the holder body 1 into which faces the through-bore 4 opens, as shown in Figs. 2 and 4, due only to the fact that the through-bore 4 is inclined with respect to the longitudinal axis X-X of the holder body 1.

A threaded hole 5 is formed in one half part 1a, extending crosswise therethrough, and a blind hole 5a is formed in the other half part 1b, extending crosswise therethrough and being aligned with the threaded hole 5.

This hole 5a has a diameter slightly greater than that of the hole 5, and therefore, a thumb screw 6 can be easily inserted through the hole 5a, and is then screwed into the threaded hole 5.

A blind pin hole 7 is formed in the one half part 1a, substantially parallel to the through-hole 5a, and a hole 7a is formed in the other half part 1b, having a diameter slightly greater than the diameter of the blind pin hole 7 and being aligned with the same.

A blade hold pin 8, for preventing the microtome blade 2 from turning around the shaft of the thumb screw 6 during use, is press-fitted in the blind pin hole 7 through the hole 7a. Although this blade hold pin 8 is a desirable safety measure, it is not always essential in the arrangement of the present invention.

Since microtome blades 2 of all standardized types have common thickness of 0.254mm and width of 8mm, the gap width G of the slit 3 is slightly larger than 0.254mm. Further, the height and the width of the front end portion of the holder body 1 are preferably about 20mm and 10mm, respectively, and the inner diameter of the through-bore 4 is set so as to allow both half parts 1a and 1b to resiliently deflect in order to clamp a microtome blade 2 inserted in the slit 3.

The microtome blade 2 shown is of the type that has chamfered corners 2b. It can be seen from Fig. 2 that because the through-bore is inclined, the chamfer of the corners 2b allows the blade to be secured in the holder with a corner edge in bearing contact with the rear wall surface 4a of the through-bore.

Next, explanation will be made hereinbelow on the use of the above-mentioned blade holder.

At first, the thumb screw 6 is loosened so as to allow the half parts 1a and 1b to resiliently deflect and open slightly to allow a used microtome blade 2 to be inserted into the slit 3. The blade 2 is placed so that its cutting edge extends along the bottom face of the holder body 1 while the top face 2a of the blade opposite to the cutting edge 2c bears against the blade hold pin 8 and the shank of the thumb screw 6. Further, the microtome blade 2 is set in the slit 3 so that the lower chamfered corner of the inserted end part of the microtome blade 2 contacts and bears against the wall surface 4a of the inclined through-bore 4 at the innermost part thereof. Then, the thumb screw 6 is tightened to clamp the microtome blade 2 between the half parts 1a and 1b.

In this condition, the microtome blade 2 projects from the front end face 9 of the holder body 1 by a substantial length sufficient for cutting paper or the like during use. Since the top face 2a of the microtome blade 2 bears against the blade hold pin 8 and the thumb screw 6 and since the lower chamfered corner 2b of the blade 2 contacts and bears against the innermost part of the wall surface 4a of the inclined through-bore 4, it is possible to surely and safely hold the blade 2 between the two halves 1a and 1b of the holder body 1 and to prevent it from being tilted even though a reaction force is placed on the tip during use.

Referring to Figs. 3 and 4, a second embodiment of the present invention will be explained. In these figures, like reference numerals are used to denote like parts to those shown in Figs. 1 and 2 so that detailed explanation thereof is abbreviated for the sake of brevity.

In the second embodiment, the front end portion of the holder body 1 is cut away along the bottom surface of the holder so as to form a cut-out which extends up to a position intermediate of the through-bore 4, leaving bifurcated bottom side projecting parts 1c and 1d in the lower opening of the through-bore 4. A blade hold pin 8' is embedded near the ends 1e and 1f of these bottom side projecting parts 1c and 1d and extends between the parts. The grip portion of the holder body 1 is provided thereon with graduations 10 so that the grip end portion of the holder body 1 can be used as a measure.

The holder in the second embodiment can be used similar to the holder in the first embodiment, excepting that a part of the cutting edge 2c of the inserted microtome blade 2 bears against the blade holder pin 8' in this second embodiment.

Referring to Fig. 5 in which a variant form of the second embodiment of the present invention is shown, the through-bore 4' is inclined in the direction reverse to that already described. In this arrangement, the upper chamfered corner of the inserted end part of the microtome blade 2 contacts and bears against the innermost part of the wall surface of the through-bore 4' while a part of the cutting edge 2c the bottom corner of the blade bears against the blade hold pin 8' embedded in the bottom side projecting parts 1e and 1f, and accordingly, it is possible to prevent the microtome blade 2 from being turned in both directions.

The holder having the arrangement as mentioned above, according to the present invention, requires few components and has a simple structure, and accordingly can be manufactured easily at a low cost. Further, the attaching of a microtome blade to the holder of this invention can be done easily and safely since the microtome blade can be stably borne against the wall surface of the inclined through-bore and the shank of the thumb screw, and the microtome blade having been attached thereto can be prevented from being turned during the use. It is thus possible to efficiently and safely reuse discarded microtome blades.

Although the preferred embodiments of the present invention have been explained, in detail, hereinabove, the present invention should not be limited to these embodiments alone, but various modifications and changes can be made thereto without departing from the scope of the invention.

## Claims

1. A holder for clamping a microtome blade comprising an elongate body (1), characterised in that a front end of the body is bifurcated by a longitudinal slit (3), the bifurcated parts (1a,1b;1c,1d) of the front end of the body are provided with means (5,6) for clamping them onto a microtome blade (2) inserted in said slit, and an inclined through-bore (4;4') extends transversely through the body and said slit terminates at said throughbore, the rear wall (4a) of said bore serving as a contacting location transverse to the body for the rear end of a blade (2) inserted in said slit.

2. A holder as set forth in claim 1, wherein said throughbore (4) is so inclined that a top corner part (2b) of the inserted microtome blade bears against the innermost part of the wall surface of said through-bore.

3. A holder as set forth in claim 1, wherein said through-bore (4') is inclined so that a bottom corner part of the inserted microtome blade bears against the innermost part of the wall surface of said through-bore.

4. A holder as set forth in claim 1, wherein said clamping means includes a thumb screw (6) extending through a hole (5a) formed in one of said bifurcated parts and screwed into a threaded hole (5) formed in the other one of said bifurcated parts.

5. A holder as set forth in claim 1, wherein a blade hold pin (8,8') is fitted between said bifurcated half parts.

6. A holder as set forth in claim 5, wherein said blade hold pin (8) is arranged to engage a top side face of the inserted microtome blade.

7. A holder as set forth in claim 5, wherein said blade hold pin (8') is arranged to engage the cutting edge of the inserted microtome blade.

8. A holder as set forth in claim 1, wherein said throughbore (4;4') has a circular cross-sectional shape.

9. A holder as set forth in claim 1, wherein graduations (10) are provided on the grip end portion of the holder body so that the grip end portion serves as a measure.

## Patentansprüche

1. Halter zum Festklemmen einer Mikrotomklinge, umfassend einen länglichen Körper (1), dadurch gekennzeichnet, daß ein vorderes Ende des Körpers durch einen Langsschlitz (3) gegabelt ist, wobei die Gabelteile (1a,1b;1c,1d) des vorderen Endes des Körpers mit Mitteln (5,6) versehen sind, um sie auf einer Mikrotomklinge (2), die in den Schlitz eingesetzt ist, festzuklemmen, und daß eine geneigte Durchgangsbohrung (4,4') sich quer durch den Körper erstreckt und der Schlitz an der Durchgangsbohrung endet, wobei die Rückwand (4a) der Bohrung als Kontaktstelle quer zum Körper für das hintere Ende einer in den Schlitz eingesetzten Klinge (2) wirkt.

2. Halter nach Anspruch 1, worin die Durchgangsbohrung (4) so geneigt ist, daß ein oberer Eckteil (2b) der eingesetzten Mikrotomklinge gegen den innersten Teil der Wandoberfläche der Durchgangsbohrung aufruht.

3. Halter nach Anspruch 1, worin die Durchgangsbohrung (4') so geneigt ist, daß ein unterer Eckteil der eingesetzten Mikrotomklinge gegen den innersten Teil der Wandoberfläche der Durchgangsbohrung aufruht.

4. Halter nach Anspruch 1, worin das Klemmittel eine Flügelschraube (6) umfaßt, die sich durch ein Loch (5a) erstreckt, das in einem der gegabelten Teile ausgebildet ist, und die in ein Gewindeloch (5) eingeschraubt ist, das im anderen der gegabelten Teile ausgebildet ist.

5. Halter nach Anspruch 1, worin ein Klingenhaltestift (8,8') zwischen den gegabelten Halbteilen eingepaßt ist.

6. Halter nach Anspruch 5, worin der Klingenhaltestift (8) so angeordnet ist, daß er an einer oberen Seitenfläche der eingesetzten Mikrotomklinge angreift.

7. Halter nach Anspruch 5, worin der Klingenhaltestift (8') so angeordnet ist, daß er an der Schneidkante der eingesetzten Mikrotomklinge angreift.

8. Halter nach Anspruch 1, worin die Durchgangsbohrung (4;4') eine kreisförmige Querschnittsform aufweist.

9. Halter nach Anspruch 1, worin Skalierungen (10) auf dem Griffendabschnitt des Halterkörpers vorgesehen sind, sodaß der Griffendabschnitt als Meßmittel dient.

## Revendications

1. Support de fixation pour lame de microtome comprenant un corps allongé (1), caractérisé en ce qu'une extrémité avant du corps est bifurquée par une fente longitudinale (3), les parties bifurquées (1a, 1b; 1c, 1d) de l'extrémité avant du corps sont pourvues de moyens (5, 6) pour les serrer sur une lame de microtome (2) insérée dans ladite fente, et en ce qu'un perçage traversant incliné (4; 4') s'étend transversalement à travers le corps et ladite fente se termine audit perçage traversant, la paroi arrière (4a) dudit perçage servant d'emplacement de contact transversal au corps pour l'extrémité arrière d'une lame (2) insérée dans ladite fente.

2. Support selon la revendication 1, dans lequel ledit perçage traversant (4) est incliné de telle sorte qu'une partie supérieure de coin (2b) de la lame de microtome insérée porte contre la partie la plus intérieure de la surface de paroi dudit perçage traversant.

3. Support selon la revendication 1, dans lequel ledit perçage traversant (4') est incliné de telle façon qu'une partie de coin inférieure de la lame de microtome insérée porte contre la partie la plus intérieure de la surface de paroi dudit perçage traversant.

4. Support selon la revendication 1, dans lequel ledit moyen de serrage comprend une vis à oreilles (6) s'étendant à travers un perçage (5a) ménagé dans l'une desdites parties bifurquées et vissée dans un trou fileté (5) ménagé dans l'autre desdites parties bifurquées.

5. Support selon la revendication 1, dans lequel une cheville de retenue de lame (8, 8') est adaptée entre lesdites demi-parties bifurquées.

6. Support selon la revendication 5, dans lequel ladite cheville de retenue de lame (8) est diposée pour être mise en prise avec une face latérale supérieure de la lame de microtome insérée.

7. Support selon la revendication 5, dans lequel ladite cheville de retenue de lame (8') est disposée pour la mise en prise avec le bord coupant de la lame de microtome insérée.

8. Support selon la revendication 1, dans lequel ledit perçage traversant (4; 4') a une forme circulaire en section transversale.

9. Support selon la revendication 1, dans lequel des graduations (10) sont prévues sur la partie d'extrémité de préhension du corps de support de façon que la partie d'extrémité de préhension sert de mesure.
